# EUROPÄISCHE PATENTSCHRIFT

(11) **EP 0 675 093 B1**
(45) Veröffentlichungstag und Bekanntmachung des Hinweises auf die Patenterteilung: **10.06.1998**
(21) Anmeldenummer: 95103744.9
(22) Anmeldetag: 15.03.1995
(51) Int. Cl.: C07C 2/82, B01J 19/00, C07C 15/24

(54) **Verfahren zur Herstellung von 4,4'-Dimethyl-1,1'-binaphthyl**
Process for preparing 4,4'-dimethyl-1,1'-binaphthyl
Procédé pour la préparation de 4,4'-diméthyle-1,1'binaphtyle

(30) Priorität: 30.03.1994 DE 4411024
(43) Veröffentlichungstag der Anmeldung: 04.10.1995
(73) Patentinhaber: HOECHST AKTIENGESELLSCHAFT, 65929 Frankfurt am Main (DE)
(72) Erfinder: Brietzke, Stephan, Dl., D-65624 Altendiez (DE); Millauer, Hans, Dr., D-65760 Eschborn (DE)

(56) Entgegenhaltungen:
- MEMOIRS OF THE NIIHAMA COLLEGE OF TECHNOLOGY SCIENCE AND ENGINEERING, Bd. 25, 1989 Seiten 58-63, K. TABUCHI ET AL. 'Electro-Oxidation of 2-Methylnaphthalene in Aqueous Acetonitrile'
- HELV. CHIM. ACTA, Bd. 11, 1928 Seiten 1264-1267, FICHTER UND HERSZBEIN 'Elektrochemische Oxidation des alpha-Methyl-naphthalins'

## Beschreibung

Die vorliegende Erfindung betrifft ein Verfahren zur Herstellung von 4,4'-Dimethyl-1,1'-binaphthyl durch elektrolytische Dimerisierung von 1-Methylnaphthalin.

4,4'-Dimethyl-1,1'-binaphthyl ist ein Zwischenprodukt für Polyamide, Polyester, optische Aufheller und Farbstoffe.

Die Herstellung von 4,4'-Dimethyl-1,1'-binaphthyl auf chemischem Wege kann prinzipiell durch reduktive Kupplung von 1-Brom-4-methylnaphthalin nach der Methode von Kumada-Tamao erfolgen (Org. Synth., Vol. 58, 127 (1978)) erfolgen. Dazu wird 1-Brom-4-methyl-naphthalin in die entsprechende Grignard-Verbindung überführt und anschließend mit 1-Brom-4-methyl-naphthalin unter Verwendung eines Nickel-haltigen Katalysators, zum Beispiel Ni (PPh₃)₂Cl₂, gekuppelt. Das Verfahren liefert als Produktionsabfall ein Gemisch von Magnesiumbromid und Nickelsalzen, die getrennt und aufgearbeitet werden müssen.

Eine oxidative Dehydrodimerisierung auf chemischem Wege wird beschrieben von McKillop et al [J. Am. Chem. Soc. 1980, 102 (21) 6504-12]. Die Reaktion verläuft zwar sehr selektiv, nachteilig ist jedoch die Verwendung von Tl(COOCF₃)₃ als Reaktand in äquimolarer (bezogen auf Produkt) Menge. Ebenfalls von Nachteil ist der Einsatz von Trifluoressigsäure (Ausbeute 85 %) als Lösemittel bzw. Tetrachlormethan (3 l/mol) Bortrifluorid-etherat (1 l/mol) (Ausbeute 93 %).

Ein weiteres oxidatives Dimerisierungsverfahren erfolgt mit Sauerstoff an Platin- oder Palladiumkohle in Trifluoressigsäure.

Eine elektrochemische Methode wird beschrieben von Fichter und Herszbein. (Helvetica Chimica Acta 1928, 11, 1265.).

Als Elektrolyt wurde in dieser Publikation ein Gemisch aus Aceton und Schwefelsäure verwendet, als Anode diente das Reaktionsgefäß aus voroxidiertem Blei, als Kathode ein Zinnrührer. Die Elektrolyselösung enthielt ca. 10 Gew.-% 1-Methylnaphthalin, wobei eine Ausbeute von 10,5 Gew.-% 4,4'-Dimethyl-1,1'-binaphthyl erreicht wurde.
Diese Methode ist wegen der erforderlichen Apparaturen und ihrer unzureichenden Effizienz für technische Anwendungen nicht geeignet.

K. Tabuchi et al.. Memoirs of the Niihama College of Technology Science and Engineering, 58 (1989) beschreiben die potentialkontrollierte Elektrooxidation von 2-Methylnaphthalin in wäßrigem Acetonitril oder Aceton.

Es bestand daher ein großer Bedarf nach einem Verfahren, das 4,4'-Dimethyl-1,1'-binaphthyl ausgehend von gut zugänglichen Edukten in hoher Ausbeute und Reinheit zugänglich macht.

Diese Aufgabe wird gelöst durch ein Verfahren zur Herstellung von 4,4'-Dimethyl-1,1'-binaphthyl, dadurch gekennzeichnet, daß 1-Methylnaphthalin in Gegenwart von Acetonitril/Wasser/Leitsalzgemischen, die mindestens noch eine weitere, mit Wasser nicht oder nur teilweise mischbare Komponente enthalten, elektrochemisch oxidativ dimerisiert wird.

Das elektrochemische Verfahren verläuft nach folgender Reaktionsgleichung:

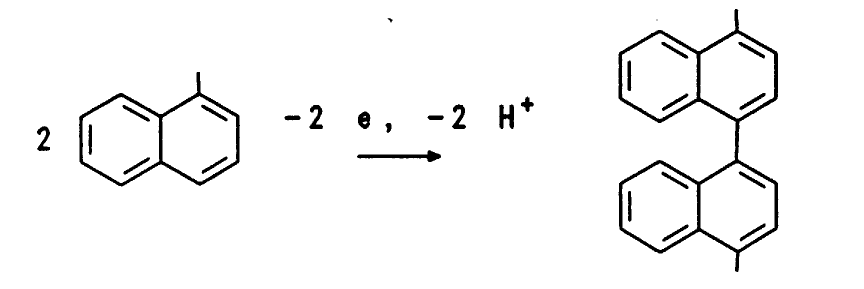

Die Reaktion verläuft bei erfindungsgemäßen Vorgehen hochselektiv, die Ausbeute beträgt etwa 50 % d. Th..

Die für das erfindungsgemäße Verfahren verwendeten Elektrolytsysteme können auf verschiedene Weise gebildet werden. Grundsätzlich sind am Aufbau solcher Elektrolytsysteme aber stets Acetonitril, Wasser, ein Leitsalz und mindestens eine weitere, mit Wasser nicht oder nur teilweise mischbare Komponente beteiligt.

Die mit Wasser nicht mischbare Komponente kann ein (a) aliphatischer oder cycloaliphatischer Kohlenwasserstoff sein, wie zum Beispiel Pentan, Hexan, Cyclohexan, Methylcyclohexan, Heptan, Oktan, Isooktan, Dekan, Dodekan oder Dekalin oder Gemische (Destillationsschnitte) solcher Verbindungen, oder (b) ein aromatischer Kohlenwasserstoff sein, wie zum Beispiel Benzol, Toluol, o-, m-, oder p-Xylol, Mesitylen, Naphthalin oder Tetralin, oder (c) ein halogenierter Kohlenwasserstoff sein, wie zum Beispiel Methylenchlorid oder Chlorbenzol, oder (d) ein Keton sein mit etwa 5 bis 10 C-Atomen sein, wie zum Beispiel Diethylketon, Methyl-t-butylketon oder Acetophenon.

Diese Komponente sollte einen Siedepunkt oberhalb der jeweiligen Reaktionstemperatur besitzen.

Vorzugsweise werden aliphatische, cycloaliphatische oder aromatische Kohlenwasserstoffe mit 6 bis 10 C-Atomen eingesetzt; besonders bevorzugt sind Heptan und Toluol.

Die mit Wasser nicht mischbare Komponente des Elektrolytsystems kann auch ein Gemisch von zwei oder mehreren der vorstehend genannten Verbindungen sein.

Als Leitsalze kommen für das erfindungsgemäße Verfahren die Alkali-, Erdalkali-, Ammonium-, Monoalkylammonium-, Dialkylkammonium-, Trialkylammonium-, oder Tetralkylammoniumsalze von Säuren in Betracht, deren komplexe Anionen sich vom 6-wertigen Schwefel, vom 5-wertigen Phosphor oder vom 3-wertigen Bor ableiten. Gemische aus verschiedenen der genannten Leitsalze sind ebenfalls verwendbar.

Eingesetzt werden können beispielsweise die Salze folgender Anionen: Hydrogensulfat, Methylsulfat, Ethylsulfat, Methansulfonat, Ethansulfonat, Propansulfonat, Butansulfonat, Oktansulfonat, Benzolsulfonat, Toluolsulfonat, 2-Chlorbenzolsulfonat, p-Chlorbenzolsulfonat, 2,4-Dichlorbenzolsulfonat, Naphthalin-1-sulfonat, Naphthalin-2-sulfonat; Methanphosphonat, Ethanphosphonat, Propanphosphonat, Butanphosphonat, Hexafluorphosphat; Tetrafluorborat; geeignet sind ferner die Salze von Alkansulfonsäuren oder Alkanphosphonsäuren, deren Alkylrest poly- oder perfluoriert ist, wie zum Beispiel Trilfuormethansulfonat, 1,1,2,3,3,3-Hexafluorpropansulfonat, Nonafluorbutansulfonat, Heptadecafluor-oktansulfonat, Trifluormethanphosphonat, Nonafluorbutanphosphonat.

Es hat sich in vielen Fällen bewährt, Natrium oder Tetraalkylammoniumsalze (mit Alkylresten von 1 bis 4 C-Atomen) der Alkansulfonsäuren (mit Alkylresten von 1 bis 8 C-Atomen oder mit poly- oder perfluorierten Alkylresten von 1 bis 4 C-Atomen), der Arylsulfonsäuren (mit Aryl = Phenyl, Naphthyl; alkyl oder chlorsubstituiertes Phenyl), der Tetrafluorborsäure und der Hexafluorphosphorsäure, als Leitsalze zu verwenden.

Bevorzugte Leitsalze sind Natrium- oder Tetraalkylammoniumtetrafluoroborat.

Das Mengenverhältnis von Acetonitril zu Wasser liegt bei den verwendeten Elektrolyten im Bereich von 100:1 bis 1:1; es hat sich als günstig erwiesen im Bereich von 20:1 bis 2:1 zu arbeiten. Die Menge der mit Wasser nicht oder nur teilweise mischbare Komponente liegt, bezogen auf das Gesamtgewicht des Elektrolyten, im Bereich von 10 bis 90 %; bevorzugt ist der Bereich von 30 bis 80 %.

Die Menge des Leitsalzes liegt, bezogen auf das Gesamtgewicht des Elektrolyten, im Bereich von 0,5 bis 15 %; bevorzugt ist der Bereich von 1 bis 7 %.

Die Mengenverhältnisse der Komponenten der Elektrolyten werden vorzugsweise so eingestellt daß sich ein zweiphasiges System ergibt, in welchem sich Wasser und Leitsalz entweder in der Acetonitrilphase befinden oder weitgehend von der organischen Phase abgetrennt als eigenständige Phase existieren, wobei unterschiedliche Anteile des Leitsalzes prinzipiell in beiden Phasen zu finden sind. Eine einphasige Reaktionsführung ist jedoch auch möglich.

Das Verfahren wird in einer ungeteilten Elektrolysezelle durchgeführt. Für größere Elektrolysen werden bevorzugt Durchflußzellen mit einem bipolar geschalteten Stapel von Elektroden benutzt. Als Anodenmaterial eignet sich Graphit, glasartiger Kohlenstoff, Platin oder Edelstahl; Edelstahl-Anoden sind bevorzugt. Das Kathodenmaterial ist nicht kritisch. Es können alle üblichen Metalle wie z. B. Stahl, Edelstahl, Nickel, Titan, Kupfer, Platin sowie Graphit oder glasartiger Kohlenstoff benutzt werden; Edelstahl ist bevorzugt.

Das erfindungsgemäße Verfahren wird bei Stromdichten im Bereich von 10 bis 250 mA/cm², vorzugsweise im Bereich von 25 bis 150 mA/cm² und besonders bevorzugt im Bereich von 40 bis 100 mA/cm² durchgeführt.

Die Elektrolyse erfolgt im allgemeinen bei Temperaturen zwischen 0 und 80°C; ein bevorzugter Temperaturbereich liegt zwischen 50 und 65°C.

Die Zielverbindung ist in den meisten Elektrolytsystemen bei Raumtemperatur nur schlecht löslich und läßt nach Beendigung der Reaktion durch Abkühlen des Reaktionsgemisches in grobkristalliner Form gewinnen und kann anschließend durch Filtration isoliert werden.

Besonders vorteilhaft an dem erfindungsgemäßen Verfahren ist, daß teure Zwischenprodukte wie 1-Brom-4-methylnaphthalin und problematische schwermetallhaltige Abfälle vermieden werden und mit einfachen Mitteln die Isolierung des Produkts, sowie die Rezyklisierung von nicht umgesetzten Rohstoff, Lösungsmitteln und Leitsalzen möglich ist.

Durch das Verfahren wird das Produkt mit verbesserten Strom- und Materialausbeuten erhalten und technisch verwertbare Umsätze erreicht.

Das Verfahren kann auch in einer Durchflußzelle durchgeführt werden.

Die folgenden Beispiele sollen das Verfahren erläutern ohne es darauf zu beschränken.

### Beispiele:

| 1. | |
|---|---|
| Edukt | 170 g 1-Methylnaphthalin (1,2 Mol) |
| Elektrolyt | 1500 ml Acetonitril / 1500 ml n-Heptan |
| Zusätze | 75 ml Methanol / 75 ml Wasser |
| Leitsalz | 15 g NaBF₄ |
| Reaktionstemperatur | 50°C |
| Zelle | Ungeteilte Labordurchlaufzelle U7 |
| Anode | Graphit |
| Kathode | Edelstahl |
| Elektrodenfläche | 200 cm² |
| Stromdichte | 50 mA/cm² |
| Übertragene Ladung | 64 Ah (200 % d. Th.) |
| Umsatz | 59 % |
| Ausbeute | 40 % |

| 2. | |
|---|---|
| Edukt | 200 g 1-Methylnaphthalin (1,4 Mol) |
| Elektrolyt | 1500 ml Acetonitril / 1500 ml n-Heptan |
| Zusätze | 100 ml Wasser |
| Leitsalz | 30 g NaBF₄ |
| Reaktionstemperatur | 55°C |
| Zelle | Ungeteilte Labordurchlaufzelle U7 |
| Anode | Edelstahl |
| Kathode | Edelstahl |
| Elektrodenfläche | 200 cm² |
| Stromdichte | 50 mA/cm² |
| Übertragene Ladung | 63 Ah (166 % d. Th.) |
| Umsatz | 75 % |
| Ausbeute | 51 % |

| 3. | |
|---|---|
| Edukt | 300 g 1-Methylnaphthalin (2,11 Mol) |
| Elektrolyt | 2000 ml Acetonitril / 500 ml Toluol (einphasig) |
| Zusätze | 200 ml Wasser |
| Leitsalz | 25 g NaBF₄ |
| Reaktionstemperatur | 60°C |
| Zelle | Ungeteilte Labordurchlaufzelle U7 |
| Anode | Graphit |
| Kathode | Edelstahl |
| Elektrodenfläche | 200 cm² |
| Stromdichte | 50 mA/cm² |
| Übertragene Ladung | 56,5 Ah (100 % d. Th.) |
| Umsatz | 34 % |
| Ausbeute | 21 % |

| 4. | |
|---|---|
| Edukt | 56,8 g 1-Methylnaphthalin (0,4 Mol) |
| Elektrolyt | 250 ml Acetonitril / 250 ml Heptan |
| Zusätze | 12 ml Wasser / 12 ml Methanol |
| Leitsalz | 5 g NaBF₄ |
| Reaktionstemperatur | 20°C |
| Zelle | Topfzelle, 500 ml ungeteilt |
| Anode | Graphit EH |
| Kathode | Edelstahlnetz |
| Elektrodenfläche | 50 cm² |
| Stromdichte | 50 mA/cm² |
| Übertragene Ladung | 10,7 Ah (100 % d. Th.) |
| Umsatz | 32 % |

| 5. | |
|---|---|
| Edukt | 28,6 g 1-Methylnaphthalin (0,2 Mol) |
| Elektrolyt | 250 ml Acetonitril / 250 ml Heptan |
| Zusätze | 12 ml Wasser |
| Leitsalz | 5 g NaBF₄ |
| Reaktionstemperatur | 52°C |
| Zelle | Topfzelle, 500 ml ungeteilt |
| Anode | Edelstahlnetz |
| Kathode | Edelstahlnetz |
| Elektrodenfläche | 50 cm² |
| Stromdichte | 50 mA/cm² |
| Übertragene Ladung | 10,7 Ah (200 % d. Th.) |
| Umsatz | 83 % |

### Aufarbeitung:

Nach dem Abkühlen wird der kristalline Feststoff aus dem Reaktionsgemisch abfiltriert und aus Aceton umkristallisiert. Schmelzpunkt: 148°C (unkorr.)

## Patentansprüche

1. Verfahren zur Herstellung von 4,4'-Dimethyl-1,1'-binaphthyl, dadurch gekennzeichnet, daß 1-Methylnaphthalin in Gegenwart von Acetonitril/Wasser/Leitsalzgemischen, die mindestens noch eine weitere, mit Wasser nicht oder nur teilweise mischbare Komponente enthalten, elektrochemisch oxidativ dimerisiert wird.

2. Verfahren nach Anspruch 1, dadurch gekennzeichnet, daß die mit Wasser nicht mischbare Komponente ein aliphatischer oder cycloaliphatischer Kohlenwasserstoff, ein aromatischer Kohlenwasserstoff, ein halogenierter Kohlenwasserstoff; ein Keton oder ein Gemisch der vorstehend aufgeführten Verbindungen ist.

3. Verfahren nach Anspruch 2, dadurch gekennzeichnet, daß der aliphatische oder cycloaliphatische Kohlenwasserstoff Pentan, Hexan, Cyclohexan, Methylcyclohexan, Heptan, Oktan, Isooktan, Dekan, Dodekan, Dekalin, insbesondere ein aliphatischer oder cycloaliphatischer Kohlenwasserstoff mit 6 bis 10 C-Atomen, bevorzugt Heptan oder Oktan ist.

4. Verfahren nach Anspruch 2, dadurch gekennzeichnet, daß der aromatische Kohlenwasserstoff Benzol, Toluol, o-, m-, p-Xylol, Mesitylen, Naphthalin oder Tetralin, insbesondere ein aromatischer Kohlenwasserstoff mit 6 bis 10 C-Atomen, bevorzugt Toluol oder Xylol ist.

5. Verfahren nach Anspruch 2, dadurch gekennzeichnet, daß der halogenierte Kohlenwasserstoff Methylenchlorid oder Chlorbenzol ist.

6. Verfahren nach Anspruch 2, dadurch gekennzeichnet, daß das Keton 5 bis 10 C-Atome enthält, insbesondere Diethylketon, Methyl-tert.-Butylketon oder Acetophenon.

7. Verfahren nach Anspruch 1, dadurch gekennzeichnet, daß die mit Wasser nicht mischbare Komponente einen Siedepunkt oberhalb der jeweiligen Reaktionstemperatur besitzt.

8. Verfahren nach mindestens einem der Ansprüche 1 bis 7, dadurch gekennzeichnet, daß als Leitsalz die Alkali, Erdalkali, Ammonium-, Monoalkylammonium, Dialkylammonium, Trialkylammonium- oder Tetraalkylammoniumsalze von Säuren, deren Anionen sich vom 6-wertigen Schwefel, 5-wertigen Phosphor oder 3-wertigen Bor ableiten oder deren Gemische eingesetzt werden.

9. Verfahren nach Anspruch 8, dadurch gekennzeichnet, daß als Anionen Hydrogensulfat, Methylsulfat, Ethylsulfat, Methansulfonat, Ethansulfonat, Propansulfonat, Butansulfonat, Oktansulfonat, Benzolsulfonat, Toluolsulfonat, 2-Chlorbenzolsulfonat, p-Chlorbenzolsulfonat, 2,4-Dichlorbenzolsulfonat, Naphthalin-1-sulfonat, Naphthalin-2-sulfonat, Methanphosphonat, Ethanphosphonat, Propanphosphonat, Butanphosphonat, Hexafluorphosphat, Tetrafluorborat eingesetzt werden.

10. Verfahren nach mindestens einem der Ansprüche 1 bis 7, dadurch gekennzeichnet, daß als Leitsalz die Salze von Alkansulfonsäuren oder Alkanphosphonsäuren, deren Alkylrest poly- oder perfluoriert ist, wie z. B. Trifluormethansulfonat, 1,1,2,3,3,3-Hexafluorpropansulfonat, Nonafluorbutansulfonat, Heptadecafluor-oktansulfonat, Trifluormethanphosphonat, Nonafluorbutanphosphonat eingesetzt werden.

11. Verfahren nach mindestens einem der Ansprüche 1 bis 7, dadurch gekennzeichnet, daß als Leitsalz Natrium oder Tetraalkylammoniumsalze mit Alkylresten von 1 bis 4 C-Atomen, der Alkansulfonsäuren mit Alkylresten von 1 bis 8 C-Atomen oder mit poly- oder perfluorierten Alkylresten von 1 bis 4 C-Atomen, der Arylsulfonsäuren mit Aryl = Phenyl, Naphthyl; alkyl- oder chlorsubstituiertes Phenyl, der Tetrafluorborsäure und der Hexafluorphosphorsäure eingesetzt werden.

12. Verfahren nach mindestens einem der Ansprüche 1 bis , dadurch gekennzeichnet, daß als Leitsalz Natrium- oder Tetraalkylammoniumtetrafluoroborat eingesetzt werden.

13. Verfahren nach mindestens einem der Ansprüche 1 bis 12, dadurch gekennzeichnet, daß das Mengenverhältnis Acetonitril zu Wasser 100:1 bis 1:1, insbesondere 20:1 bis 2:1 beträgt.

14. Verfahren nach mindestens einem der Ansprüche 1 bis 13, dadurch gekennzeichnet, daß die Menge der mit Wasser nicht oder nur teilweise mischbaren Komponente 10 bis 90 Gew.-%, insbesondere 30 bis 80 Gew.-% bezogen auf das Gesamtgewicht des Elektrolyten beträgt.

15. Verfahren nach mindestens einem der Ansprüche 1 bis 14, dadurch gekennzeichnet, daß die Menge des Leitsalzes 0,5 bis 15 Gew.-%, insbesondere 1 bis 7 Gew.-% bezogen auf das Gesamtgewicht der Elektrolyten beträgt.

16. Verfahren nach mindestens einem der Ansprüche 1 bis 15, dadurch gekennzeichnet, daß die Elektrolyse bei Stromdichten von 10 bis 250 mA/cm², insbesondere 25 bis 150 mA/cm², bevorzugt 40 bis 100 mA/cm² durchgeführt wird.

17. Verfahren nach mindestens einem der Ansprüche 1 bis 16, dadurch gekennzeichnet, daß die Elektrolyse bei Temperaturen von 0 bis 80°C, insbesondere 50 bis 65°C, durchgeführt wird.

18. Verfahren nach mindestens einem der Ansprüche 1 bis 17, dadurch gekennzeichnet, daß als Anode Graphit, glasartiger Kohlenstoff, Platin oder Edelstahl, insbesondere Edelstahl verwendet wird.

19. Verfahren nach mindestens einem der Ansprüche 1 bis 18, dadurch gekennzeichnet, daß als Kathode Graphit, glasartiger Kohlenstoff, Metalle, insbesondere Edelstahl verwendet wird.

## Claims

1. A process for preparing 4,4'-dimethyl-1,1'-binaphthyl, which comprises oxidatively dimerizing 1-methylnaphthalene electrochemically in the presence of acetonitrile/water/conductivity salt mixtures comprising at least one further component which is immiscible or only partially miscible with water.

2. The process as claimed in claim 1, wherein the component which is immiscible with water is an aliphatic or cycloaliphatic hydrocarbon, an aromatic hydrocarbon, a halogenated hydrocarbon, a ketone or a mixture of the abovementioned compounds.

3. The process as claimed in claim 2, wherein the aliphatic or cycloaliphatic hydrocarbon is pentane, hexane, cyclohexane, methylcyclohexane, heptane, octane, isooctane, decane, dodecane, decalin, in particular an aliphatic or cycloaliphatic hydrocarbon having from 6 to 10 carbon atoms, preferably heptane or octane.

4. The process as claimed in claim 2, wherein the aromatic hydrocarbon is benzene, toluene, o-, m-, p-xylene, mesitylene, naphthalene or tetralin, in particular an aromatic hydrocarbon having from 6 to 10 carbon atoms, preferably toluene or xylene.

5. The process as claimed in claim 2, wherein the halogenated hydrocarbon is methylene chloride or chlorobenzene.

6. The process as claimed in claim 2, wherein the ketone contains from 5 to 10 carbon atoms, in particular diethyl ketone, methyl tert-butyl ketone or acetophenone.

7. The process as claimed in claim 1, wherein the component which is immiscible with water has a boiling point above the respective reaction temperature.

8. The process as claimed in at least one of claims 1 to 7, wherein the conductivity salts used are the alkali metal, alkaline earth metal, ammonium, monoalkylammonium, dialkylammonium, trialkylammonium or tetraalkylammonium salts of acids whose anions are derived from hexavalent sulfur, pentavalent phosphorus or trivalent boron, or mixtures thereof.

9. The process as claimed in claim 8, wherein the anions used are hydrogensulfate, methylsulfate, ethylsulfate, methanesulfonate, ethanesulfonate, propanesulfonate, butanesulfonate, octanesulfonate, benzenesulfonate, toluenesulfonate, 2-chlorobenzenesulfonate, p-chlorobenzenesulfonate, 2,4-dichlorobenzenesulfonate, naphthalene-1-sulfonate, naphthalene-2-sulfonate, methanephosphonate, ethanephosphonate, propanephosphonate, butanephosphonate, hexafluorophosphate, tetrafluoroborate.

10. The process as claimed in at least one of claims 1 to 7, wherein the conductivity salts used are the salts of alkanesulfonic acids or alkanephosphonic acids whose alkyl radical is polyfluorinated or perfluorinated, for example trifluoromethanesulfonate, 1,1,2,3,3,3-hexafluoropropanesulfonate, nonafluorobutanesulfonate, heptadecafluorooctanesulfonate, trifluoromethanephosphonate, nonafluorobutanephosphonate.

11. The process as claimed in at least one of claims 1 to 7, wherein the conductivity salts used are sodium salts or tetraalkylammonium salts having alkyl radicals containing from 1 to 4 carbon atoms, of alkanesulfonic acids having alkyl radicals containing from 1 to 8 carbon atoms or having polyfluorinated or perfluorinated alkyl radicals containing from 1 to 4 carbon atoms, of arylsulfonic acids where aryl = phenyl, naphthyl; alkyl- or chloro-substituted phenyl, of tetrafluoroboric acid and of hexafluorophosphoric acid.

12. The process as claimed in at least one of claims 1 to [lacuna], wherein the conductivity salt used is sodium or tetraalkylammonium tetrafluoroborate.

13. The process as claimed in at least one of claims 1 to 12, wherein the ratio of acetonitrile to water is from 100:1 to 1:1, in particular from 20:1 to 2:1.

14. The process as claimed in at least one of claims 1 to 13, wherein the amount of the component which is immiscible or only partially miscible with water is from 10 to 90% by weight, in particular from 30 to 80% by weight, based on the total weight of the electrolyte.

15. The process as claimed in at least one of claims 1 to 14, wherein the amount of the conductivity salt is from 0.5 to 15% by weight, in particular from 1 to 7% by weight, based on the total weight of the electrolyte.

16. The process as claimed in at least one of claims 1 to 15, wherein the electrolysis is carried out at current densities of from 10 to 250 mA/cm², in particular from 25 to 150 mA/cm², preferably from 40 to 100 mA/cm².

17. The process as claimed in at least one of claims 1 to 16, wherein the electrolysis is carried out at temperatures of from 0 to 80°C, in particular from 50 to 65°C.

18. The process as claimed in at least one of claims 1 to 17, wherein the anode used is graphite, vitreous carbon, platinum or stainless steel, in particular stainless steel.

19. The process as claimed in at least one of claims 1 to 18, wherein the cathode used is graphite, vitreous carbon, metals, in particular stainless steel.

## Revendications

1. Procédé pour la préparation du 4,4'-diméthyl-1,1'-binaphtyle, caractérisé en ce que l'on dimérise par oxydation par voie électrochimique le 1-méthylnaphtalène en présence de mélanges d'acétonitrile/eau/sel conducteur, qui contiennent encore un autre constituant non miscible ou seulement partiellement miscible à l'eau.

2. Procédé selon la revendication 1, caractérisé en ce que le constituant non miscible à l'eau est un hydrocarbure aliphatique ou cycloaliphatique, un hydrocarbure aromatique, un hydrocarbure halogéné ; une cétone ou un mélange des composés précités.

3. Procédé selon la revendication 2, caractérisé en ce que l'hydrocarbure aliphatique ou cycloaliphatique est le pentane, l'hexane, le cyclohexane, le méthylcyclohexane, l'heptane, l'octane, l'isooctane, le décane, le dodécane ou la décaline, plus particulièrement un hydrocarbure aliphatique ou cycloaliphatique avec 6 à 10 atomes de carbone, de préférence l'heptane ou l'octane.

4. Procédé selon la revendication 2, caractérisé en ce que l'hydrocarbure aromatique est le benzène, le toluène, l'o-, le m-, le p-xylène, le mésitylène, le naphtalène ou la tétraline, plus particulièrement un hydrocarbure aromatique avec 6 à 10 atomes de carbone, de préférence le toluène ou le xylène.

5. Procédé selon la revendication 2, caractérisé en ce que l'hydrocarbure halogéné est le chlorure de méthylène ou le chlorobenzène.

6. Procédé selon la revendication 2, caractérisé en ce que la cétone comporte de 5 à 10 atomes de carbone, et il s'agit plus particulièrement de la diéthylcétone, de la méthyl-tert.-butylcétone ou de l'acétophénone.

7. Procédé selon la revendication 1, caractérisé en ce que le composant non miscible à l'eau présente un point d'ébullition supérieur à la température réactionnelle respective.

8. Procédé selon au moins l'une des revendications 1 à 7, caractérisé en ce que l'on utilise comme sel conducteur des sels d'acide avec des alcalis, des métaux alcalino-terreux, l'ammonium, le monoalkylammonium, le dialkylammonium, le trialkylammonium ou le tétraalkylammonium, dont les anions dérivent du soufre hexavalent, du phosphore pentavalent ou du bore trivalent, ou leurs mélanges.

9. Procédé selon la revendication 8, caractérisé en ce que l'on utilise en tant qu'anions les suivants : bisulfate, sulfate de méthyle, sulfate d'l'éthyle, méthanesulfonate, éthanesulfonate, propanesulfonate, butanesulfonate, octanesulfonate, benzènesulfonate, toluènesulfonate, 2-chlorobenzènesulfonate, p-chlorobenzènesulfonate, 2,4-dichlorobenzènesulfonate, naphtalène-l-sulfonate, naphtalène-2-sulfonate, méthanephosphonate, éthanephosphonate, propanephosphonate, butanephosphonate, hexafluorophosphate ou tétrafluoroborate.

10. Procédé selon au moins l'une des revendications 1 à 7, caractérisé en ce que l'on utilise en tant que sels conducteurs des sels d'acide alcanesulfonique ou d'acide alcanephosphonique, dont les restes alkyle sont poly- ou perfluorés, comme par exemple le trifluorométhanesulfonate, le 1,1,2,3,3,3-hexafluoropropanesulfonate, le nonafluorobutanesulfonate, l'heptadécafluorooctanesulfonate, le trifluorométhanephosphonate ou le nonafluorobutanephosaphonate.

11. Procédé selon au moins l'une des revendications 1 à 7, caractérisé en ce que l'on utilise en tant que sel conducteur des sels de sodium ou de tétralkylammonium, avec des restes alkyle comportant de 1 à 4 atomes de carbone, des acides alcanesulfoniques avec des restes alkyle comportant de 1 à 8 atomes de carbone ou des restes alkyle avec de 1 à 4 atomes de carbone poly- ou perfluorés, des acides arylsulfoniques avec aryle = phényle, naphtyle ; phényle substitué par alkyle ou par le chlore, d'acide tétrafluoroborique et d'acide hexafluorophosphorique.

12. Procédé selon au moins l'une des revendications 1 à 11, caractérisé en ce que l'on utilise en tant que sel conducteur le tétrafluoroborate de sodium ou de tétraalkylammonium.

13. Procédé selon au moins l'une des revendications 1 à 12, caractérisé en ce que le rapport quantitatif de l'acétonitrile à l'eau est de 100:1 à 1:1, plus particulièrement de 20:1 à 2:1.

14. Procédé selon au moins l'une des revendications 1 à 13, caractérisé en ce que la quantité du composant non miscible ou seulement partiellement miscible à l'eau est de 10 à 90 % en poids, plus particulièrement de 30 à 80 % en poids par rapport au poids total de l'électrolyte.

15. Procédé selon au moins l'une des revendications 1 à 14, caractérisé en ce que la quantité du sel conducteur est de 0,5 à 15 % en poids, plus particulièrement de 1 à 7 % en poids par rapport au poids total de l'électrolyte.

16. Procédé selon au moins l'une des revendications 1 à 15, caractérisé en ce que l'on met en oeuvre l'électrolyse avec des densités de courant de 10 à 250 mA/cm², plus particulièrement de 25 à 150 mA/cm², de manière particulièrement préférée de 40 à 100 mA/cm².

17. Procédé selon au moins l'une des revendications 1 à 16, caractérisé en ce que l'on met en oeuvre l'électrolyse à des températures de 0 à 80 °C, plus particulièrement de 50 à 65 °C.

18. Procédé selon au moins l'une des revendications 1 à 17, caractérisé en ce que l'on utilise en tant qu'anode le graphite, le carbone vitrifié, le platine ou l'acier inoxydable, plus particulièrement l'acier inoxydable.

19. Procédé selon au moins l'une des revendications 1 à 18, caractérisé en ce que l'on utilise en tant que cathode le graphite, le carbone vitrifié, des métaux, plus particulièrement l'acier inoxydable.
